# EUROPÄISCHE PATENTANMELDUNG

(11) **EP 0 959 090 A1**
(43) Veröffentlichungstag der Anmeldung: **24.11.1999**
(21) Anmeldenummer: 99109049.9
(22) Anmeldetag: 07.05.1999
(51) Int. Cl.: C08G 73/10, C08G 73/16, C08G 73/02, A61K 7/48, A61K 7/06, C11D 3/37

(54) **Copolymere, hydrophob modifizierte Polyasparaginsäureester mit erhöhter Molekularmasse und deren Verwendung**

(30) Priorität: 20.05.1998 DE 19822600
(71) Anmelder: Th. Goldschmidt AG, 45127 Essen (DE)
(72) Erfinder: Grüning, Burghard Dr., 45134 Essen (DE); Simpelkamp, Jörg Dr., 45130 Essen (DE); Weitemeyer, Christian Dr., 45134 Essen (DE)

(57) **Zusammenfassung**

Die Erfindung beschreibt die Herstellung hochmolekularer copolymerer Polyasparaginsäureester, welche mit Alkylresten mit 6 bis 30 C-Atomen hydrophob modifiziert sind.

Von Polyaminosäuren abgeleitete Copolymere, die zu mindestens 75 Mol-% der vorhandenen Einheiten aus Struktureinheiten der allgemeinen Formeln (I), (II) oder (III) 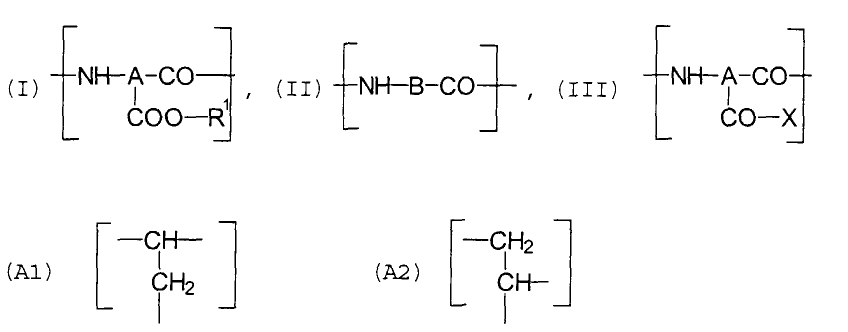 bestehen, in denen die Strukturelemente A gleiche oder verschiedene trifunktionelle Kohlenwasserstoffradikale mit 2 C-Atomen des Typs (A1) oder (A2) sind, wobei ein Copolymeres aus mindestens drei Einheiten der Formel (I) besteht, worin
R¹ die Bedeutung von R², R³ oder R⁴ hat, wobei
R² ein oder mehrere Reste aus der Gruppe der Alkali, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Alkenyl mit 1 bis 22 C-Atomen oder Hydroxyalkyl mit 1 bis 22 C-Atomen mit 1 bis 6 Hydroxygruppen und/oder deren Acylierungsprodukte mit C₁- bis C₂₂-Carbonsäureresten bedeuten,
R³ gleiche oder verschiedene, geradkettige oder verzweigte, gesättigte oder ungesättigte Alkyl- oder Alkenylreste R⁹ mit 6 bis 30 C-Atomen oder Radikale der Struktur -Y-R⁹ sind, wobei Y eine Oligo- oder Polyoxyalkylenkette mit 1 bis 100 Oxyalkyleneinheiten ist,
R⁴ gleiche oder verschiedene, geradkettige oder verzweigte, gesättigte oder ungesättigte Alkyl- oder Alkenylreste mit 1 bis 5 C-Atomen sind,
   die Einheiten der Formel (II) für proteinogene oder nicht proteinogene Aminosäuren stehen und zu nicht mehr als 20 Gew.-% enthalten sind und
X in der Formel (III) für ein oder mehrere di- oder polyfunktionelle Radikale abgeleitet von Molekularmassen erhöhenden Agenzien, insbesondere einer Di- oder Polyhdroxyverbindung, einer Di- oder Polyaminoverbindung, oder Aminoalkoholen, mit einem linearen, verzweigten oder cyclischen, gesättigten, ungesättigten oder aromatischen Kohlenwasserstoffgerüst, gegebenenfalls oxo- oder azasubstituiert mit O- oder N-Atomen in der Kette, steht,
   und mindestens jeweils ein Rest R¹ die Bedeutung von R² und mindestens ein Rest R¹ die von R³ und wenigstens ein Rest R¹ die von X annehmen muß.

## Beschreibung

Diese Erfindung beschreibt die Herstellung hochmolekularer copolymerer Polyasparaginsäureester, welche mit Alkyl- oder Alkenylresten mit 6 bis 30 C-Atomen hydrophob modifiziert sind.

Polyaminosäurederivate, insbesondere Polyasparaginsäure, haben in jüngster Zeit aufgrund ihrer biologischen Abbaubarkeit und Naturnähe besondere Aufmerksamkeit gefunden. Es werden unter anderem Anwendungen als biologisch abbaubare Komplexierungsmittel, Enthärter und Waschmittel-Builder vorgeschlagen. Polyasparaginsäure wird i.A. durch alkalische Hydrolyse der unmittelbaren Synthesevorstufe Polysuccinimid (PSI, Anhydropolyasparaginsäure), dem cyclischen Imid der Polyasparaginsäure gewonnen. PSI kann beispielsweise nach EP 0 578 449 A, WO 92/14753, EP 0 659 875 A oder DE 44 20 642 A aus Asparaginsäure hergestellt werden oder ist beispielsweise nach DE 36 26 672 A, EP 0 612 784 A, DE 43 00 020 A oder US 5 219 952 A aus Maleinsäurederivaten und Ammoniak zugänglich. Für diese üblichen Polyasparaginsäuren werden unter anderem Anwendungen als Inkrustationsinhibitor, Builder in Waschmitteln, Düngemitteladditiv und Hilfsstoff in der Gerberei vorgeschlagen.

Die von verschiedenen Arbeitsgruppen beschriebene Umsetzung von Polysuccinimid mit Aminen führt zu Polyasparaginsäureamiden (Kovacs et al., J. Med. Chem. 1967, 10, 904-7; Neuse, Angew. Makromol. Chem. 1991, 192, 35-50). Die Ringöffnung von Polysuccinimid mit Polyaminen und die nachfolgende alkalische Hydrolyse zur Herstellung von Polyasparaginsäurederivaten für Anwendungen als Superabsorber wird beispielsweise in WO 95/35337, in WO 96/08523 oder in Annu. Tech. Conf. Soc. Plast. Eng.1995, 53, 1510-13 beschrieben. Neri et al beschreiben in J. Med. Chem 1973, 16, 893-897 die Umsetzung von Polysuccinimid mit Ethanolamin zu Hydroxyethyl-Polyaspartaten für pharmazeutische Anwendungen.

Von besonderem Interesse unter anderem für Anwendungen als Emulgator, Dispergiermittel und Tensid sind copolymere Polyasparaginsäureester, welche partiell mit langkettigen Fettalkoholen oder deren Derivaten verestert sind. Derartige Verbindungen sind auf Basis von Maleinsäuremonoestern und Ammoniak leicht zugänglich, wie aus DE 195 45 678 beziehungsweise der EP 96 118 806.7 hervorgeht, und weisen im allgemeinen eher niedrige Molekulargewichte auf.

Aufgabe der Erfindung war die Bereitstellung copolymerer Polyasparaginsäureester mit erhöhten Molekularmassen.

Die Aufgabe wird erfindungsgemäß gelöst durch copolymere Polyasparaginsäureester mit erhöhter Molekularmasse, die aus Maleinsäurederivaten und Ammoniak unter Zusatz von di- oder polyfunktionellen Alkoholen oder Aminen hergestellt werden.

Die eingesetzten, von Polyasparaginsäure abgeleiteten Copolymeren bestehen zu wenigstens 75 Mol-% der vorhandenen Einheiten aus Struktureinheiten der allgemeinen Formeln (I), (II) und (III) 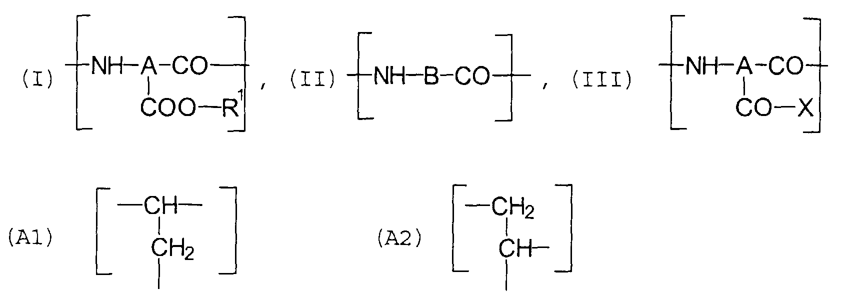 in denen die Strukturelemente A gleiche oder verschiedene trifunktionelle Kohlenwasserstoffradikale mit 2 C-Atomen des Typs (A1) oder (A2) sind, wobei ein Copolymeres aus mindestens drei Einheiten der Formel (I) besteht, worin
R¹ die Bedeutung von R², R³ oder R⁴ hat, wobei
R² ein oder mehrere Reste aus der Gruppe der Alkali, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Alkenyl mit 1 bis 22 C-Atomen oder Hydroxyalkyl mit 1 bis 22 C-Atomen und 1 bis 6 Hydroxygruppen und/oder deren Acylierungsprodukte mit C₁ bis C₂₂-Carbonsäureresten bedeuten,
R³ gleiche oder verschiedene, geradkettige oder verzweigte, gesättigte oder ungesättigte Alkyl- oder Alkenylreste R⁹ mit 6 bis 30 C-Atomen oder Radikale der Struktur -Y-R⁹ sind, wobei Y eine Oligo- oder Polyoxyalkylenkette mit 1 bis 100 Oxyalkyleneinheiten ist,
R⁴ gleiche oder verschiedene, geradkettige oder verzweigte, gesättigte oder ungesättigte Alkyl- oder Alkenylreste mit 1 bis 5 C-Atomen sind,
   die Einheiten der Formel (II) für proteinogene oder nichtproteinogene Aminosäuren stehen und zu nicht mehr als 20 Gew.-%, bezogen auf die copolymeren Polyasparaginsäurederivate, enthalten sind und

X in der Formel (III) für ein oder mehrere di- oder polyfunktionelle Radikale abgeleitet von Molekularmassen erhöhenden Agenzien, insbesondere einer Di- oder Polyhydroxyverbindung, einer Di- oder Polyaminoverbindung, oder Aminoalkoholen, mit einem linearen, verzweigten oder cyclischen, gesättigten, ungesättigten oder aromatischen Kohlenwasserstoffgerüst, gegebenenfalls oxo- oder azasubstituiert mit O- oder N-Atomen in der Kette, steht, und mindestens jeweils ein Rest R¹ die Bedeutung von R² und mindestens ein Rest R¹ die von R³ und wenigstens ein Rest R¹ die von X annehmen muß.

Alle gegebenen Angaben zur Zusammensetzung der polymeren Produkte beziehen sich wie üblich auf die mittlere Zusammensetzung der Polymerketten.

Die restlichen Einheiten, welche nicht die Struktur (I) oder (II) oder (III) haben (nicht mehr als 25 Mol-% aller Einheiten), können unter anderem Iminodisuccinat-Einheiten der allgemeinen Formel (IV) 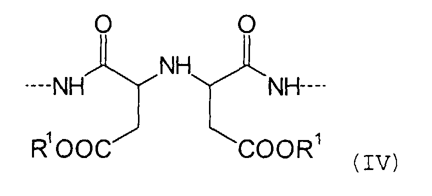 sowie verschiedene Endgruppen sein, am N-Terminus beispielsweise Asparaginsäure-, Maleinsäure-, Fumarsäure- und Äpfelsäureeinheiten sowie deren Ester oder Amide, Maleinimideinheiten oder Diketopiperazine abgeleitet von Asparaginsäure und/oder den Aminosäurebausteinen (II), sowie Ester oder Amide der Bausteine (II), am C-Terminus beispielsweise Asparagin- oder Äpfelsäureeinheiten, deren Mono- oder Diester, Amide oder cyclischen Imide.

Als Aminosäurebausteine (II) aus der Gruppe der proteinogenen Aminosäuren kommen z. B. Glutamin, Asparagin, Lysin, Alanin, Glycin, Tyrosin, Tryptophan, Serin und Cystein sowie deren Derivate in Frage; nicht proteinogene Aminosäuren können beispielsweise β-Alanin, ω-Amino-1-alkansäuren, beispielsweise 6-Amino-capronsäure, etc. sein.

Überraschenderweise weisen diese Derivate in ihren anwendungstechnischen Eigenschaften deutliche Vorzüge gegenüber den analogen Verbindungen mit geringerem Molekulargewicht auf, insbesondere bezüglich der Temperatur- und Langzeitstabilität der Zubereitungen im Bereich kosmetischer W/O und O/W-Emulsionen sowie für Pigmentdispersionen für Lacke und Farben.

Erfindungsgemäß bevorzugt sind Verbindungen, bei denen wenigstens eine freie Carboxylatgruppe (R¹ = H, Metall, Ammonium) vorhanden ist, wenigstens ein Rest R³ gleiche oder verschiedene Radikale der Struktur R⁹-Y- umfaßt, wobei R⁹ aus der Gruppe der geradkettigen oder verzweigten, gesättigten oder ungesättigten Alkyl- oder Alkenylreste mit 6 bis 30 C-Atomen stammt (beispielsweise verzweigte oder lineare Octyl-, Decyl-, Dodecyl-, Tetradecyl-, Hexadecyl-, Octadecyl-, Docosylreste, auch ungesättigte und mehrfach ungesättigte Spezies wie beispielsweise Oleyl) und Y eine Polyoxyalkylenkette von 0 bis 100 Alkylenglykoleinheiten, vorzugsweise abgeleitet von Ethylenoxid, Propylenoxid oder Gemischen daraus und gegebenenfalls ein Rest R⁴ aus der Gruppe der geradkettigen oder verzweigten, gesättigten oder ungesättigten Alkyl- oder Alkenylreste mit 1 bis 5 C-Atomen stammt (beispielsweise Methyl, Ethyl, n-Propyl, i-Propyl, n-Butyl, i-Butyl, s-Butyl, n-Pentyl) sowie wenigstens ein polyfunktioneller Rest X, bevorzugt abgeleitet von einer gesättigten Di- oder Polyhydroxy- oder einer Di- oder Polyaminoverbindung wie beispielsweise linearen 1,ω-Alkandiolen, Glycerin, Sorbitol, 1,2-Propylenglykol, linearen 1,ω-Diaminoalkanen, Lysin, Ethanolamin, Diethanolamin, Triethanolamin, Zuckerderivaten, Oligo- und Polysacchariden, sowie den Anlagerungsprodukten der genannten Verbindungen mit Ethylenoxid und/oder Propylenoxid. Weitere geeignete polyfunktionelle Verbindungen sind beispielsweise Polyvinylalkohol, Oligo- und Polyethylenglykole oder Ethylenoxid-Propylenoxid-Copolymeren.

Eine bevorzugte Form der Copolymeren enthält Alkyl- oder Alkenylreste R⁹ mit 10 bis 22 C-Atomen ohne Alkylenglykolspacer (Alkylenglykolkettenlänge 0), vernetzende Gruppen (III) abgeleitet von 1,ω-Diolen und Diaminen mit 4 bis 6 C-Atomen, Polyethylenglykolen mit einer Molekularmasse von 200 bis 2000 oder Mono-, Di- beziehungsweise Triethanolamin, sowie gegebenenfalls geringe Mengen von Alkyl- oder Alkenylresten R⁴ mit 1 bis 4 C-Atomen.

Diese Derivate sind beispielsweise durch ein Herstellverfahren zugänglich, das dadurch gekennzeichnet ist, daß man ein Gemisch von Monoestern monoethylenisch ungesättigter Dicarbonsäuren mit 0,1 bis 3,0 Äquivalenten Ammoniak, vorzugsweise mit 0,8 bis 1,5 Äquivalenten an Ammoniak, umsetzt bzw. die Ammoniumsalze dieser Säuren thermisch in das Polymer überführt. Eingesetzt werden können beispielsweise Derivate der Maleinsäure, Fumarsäure, Itaconsäure, Alkenylbernsteinsäure, Alkylmaleinsäure, Citraconsäure oder deren Ammoniumsalze, vorzugsweise Derivate der Maleinsäure, Fumarsäure oder Itaconsäure, besonders vorzugsweise Maleinsäurederivate der allgemeinen Formeln (V) und (VI) 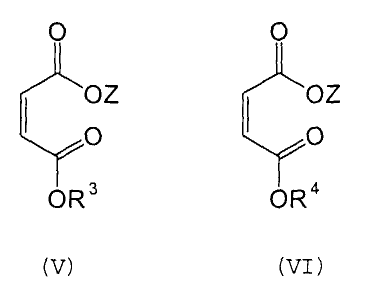 wobei Z für Wasserstoff oder Ammonium, R³ und R⁴ für die oben genannten Reste stehen. Diese Maleinsäurederivate können jeweils alleine oder in beliebigen Gemischen miteinander eingesetzt werden.

Vorzugsweise eingesetzte Reste R³ sind Alkylreste mit 8 bis 30 C-Atomen, beispielsweise lineare oder verzweigte Decyl-, Dodecyl-, Tetradecyl-, Hexadecyl-, oder Octadecylreste sowie ungesättigte Alkenylreste, wie beispielsweise Oleyl. Vorzugsweise eingesetzte Reste R⁴ sind Alkylreste mit 1 bis 4 C-Atomen, vorzugsweise Methyl, Ethyl, n-Propyl, i-Propyl, n-Butyl, i-Butyl, s-Butyl.

Die Reaktion kann mit oder ohne Zusatz von organischen Lösungsmitteln erfolgen. Als Lösungsmittel kommen beispielsweise Alkohole, Ketone, Ester, Oligo- und Poly(alkylen)glykole beziehungsweise -glykolether, Dimethylsulfoxid, Dimethylformamid, N,N-Dimethylacetamid oder N-Methylpyrrolidon sowie deren Gemische und andere in Frage. Bevorzugt eingesetzt werden Alkohole mit 2 bis 4 C-Atomen, davon besonders bevorzugt der kurzkettige Alkohol R⁴OH, sowie Ketone wie z. B. Methylisobutylketon oder Methylisoamylketon oder Alkylester von Carbonsäuren mit 1 bis 4 C-Atomen, wie beispielsweise Essigsäure-sec-butylester oder Essigsäurepentylester. Die Reaktion kann optional in Gegenwart von verträglichkeitsfördernden Agenzien durchgeführt werden. Dieses können grenzflächenaktive Verbindungen sein, beispielsweise Anlagerungsprodukte von 1 bis 30 Mol Ethylenoxid und/oder 0 bis 5 Mol Propylenoxid an C₁₂-C₃₀-Fettalkohole und Wollwachsalkohole; Ethylenoxidanlagerungsprodukte von Glycerinmono- und -diestern und Sorbitanmono- und -diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäurepartialester von Anlagerungsprodukten von 1 bis 30 Mol Ethylenoxid an Glycerin; Anlagerungsprodukte von Ethylenoxid an Fette und Öle, beispielsweise Rizinusöl oder gehärtetes Rizinusöl; Partialester von gesättigten oder ungesättigten C₁₂-C₂₂-Fettsäuren, auch verzweigte oder hydroxysubstituierte, mit Polyolen, beispielsweise Ester von Glycerin, Ethylenglykol, Polyalkylenglykolen, Pentaerythrit, Polyglycerin, Zuckeralkoholen wie Sorbit und Polyglucosiden wie Cellulose; Polysiloxan-Polyalkyl-Polyether-Copolymere und deren Derivate sowie hydrophob modifizierte Polyasparaginsäurederivate, beispielsweise teilveresterte Polyasparaginsäuren, teilveresterte Polyasparaginsäure-co-Glutaminsäuren oder Kondensate aus Maleinsäuremonoestern und Ammoniak, beispielsweise hergestellt nach dem erfindungsgemäßen Verfahren oder nach DE 195 45 678 A, wobei das Herstellverfahren der genannten Polyaminosäurederivate keinen Einfluß auf deren verträglichkeitsvermittelnde Wirkung hat. Gegebenenfalls kann auch ein gewisser Teil der Produktmischung im Reaktor verbleiben und als Lösungsvermittler für eine folgende Umsetzung dienen.

Als verträglichkeits- bzw. löslichkeitsvermittelnde Agenzien können auch kationische Tenside, beispielsweise aus der Gruppe der quarternären Ammoniumverbindungen, quarternisierten Proteinhydrolisate, Alkylamidoamine, quarternären Esterverbindungen, quarternären Siloconöle oder quarternären Zucker- und Polysaccharidderivate, anionische Tenside beispielsweise aus der Gruppe der Sulfate, Sulfonate, Carboxylate sowie Mischungen derselben, beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate wie N-Acylaspartate oder N-Acylglutamate, N-Acylsarcosinate, amphotere oder zwitterionische Tenside wie beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate, oder nichtionische Tenside wie beispielsweise oxethylierte Fettalkohole, oxethylierte Alkylphenole, oxethylierte Fettsäureester, oxethylierte Mono-, Di- oder Triglyceride oder Polyalkylenglykolfettsäureesuer, Zuckerester, z. B. Fettsäureester der Saccharose, Fructose oder des Methylglucosids, Sorbitolfettsäureester und Sorbitanfettsäureester (ggf. oxethyliert), Alkyl- oder Alkenylpolyglucoside und deren Ethoxylate, Fettsäure-N-alkylpolyhydroxyalkylamide, Polyglycerinester, Fettsäurealkanolamide, langkettige tertiäre Aminoxide oder Phosphinoxide sowie Dialkylsulfoxide enthalten sein.

Vorzugsweise verbleiben die verträglichkeitsfördernden Agenzien im Produkt. Die Umsetzung zum Copolymeren erfolgt in einer bevorzugten Ausführungsweise mit wäßrigem oder gasförmigem Ammoniak bei Temperaturen von 0 bis 150 °C, vorzugsweise 50 bis 140 °C sowie nachfolgendem Ausdestillieren bei 70 bis 240 °C, vorzugsweise 110 bis 150 °C, unter vermindertem Druck, beispielsweise in Knetapparaturen, Hochviskosreaktoren, Extrudern oder Rührreaktoren, gegebenenfalls unter Einsatz scherkraftreicher Rührer wie Mig- oder Intermig-Rührer.

Unter den Reaktionsbedingungen wird gleichzeitig ein Teil der Estergruppen, bevorzugt die von R⁴OH abgeleiteten, hydrolysiert und die gewünschten Carbonsäure- bzw. Carboxylatgruppen freigesetzt. Durch nachfolgende milde partielle oder vollständige Hydrolyse, bevorzugt der vom kurzkettigen Alkohol R⁴OH abgeleiteten Esterfunktionen kann, wenn gewünscht, der Anteil freier Säuregruppen weiter erhöht werden, beispielsweise durch Umsetzung mit Wasser gegebenenfalls in Gegenwart von Säuren oder Basen, oder mit Alkalimetallhydroxiden, gegebenenfalls in Gegenwart eines organischen Solvens oder Cosolvens.

Durch Zusatz von amino- und carboxyfunktionellen Verbindungen zur Reaktionsmischung können Copolymere erhalten werden, in denen die angebotenen Bausteine über Amidbindungen gebunden vorliegen. Geeignete Bausteine sind Aminosäuren aus der Gruppe der 20 proteinogenen Aminosäuren, welche als Monomere in allen natürlichen Proteinen enthalten sind, in enantiomerenreiner oder racemischer Form, wie beispielsweise Glutamin, Asparagin, Lysin, Alanin, Glycin, Tyrosin, Tryptophan, Serin und Cystein sowie deren Derivate, oder nicht proteinogenen Aminosäuren mit jeweils einer oder mehreren Amino- bzw. Carboxyfunktionen, wie beispielsweise β-Alanin, ω-Amino-1-alkansäuren, beispielsweise 6-Aminocapronsäure. Die Bausteine, vorzugsweise 0 bis 15 Gew.-% werden der Ausgangsmischung der Maleinsäurederivate zugesetzt oder zur Modifizierung der Kettenenden nach erfolgter Synthese des Polymeren mit diesem umgesetzt, vorzugsweise unter Zusatz polarer Solventien, wie beispielsweise Alkoholen oder Dimethylformamid.

Die Einführung der Molekularmassen erhöhenden Gruppen (IV) erfolgt durch Zusatz der polyfunktionellen Amino- bzw. Hydroxyverbindungen zur Reaktionsmischung. Die Zugabe kann vor, während oder nach der Neutralisation der Maleinsäuremonoester mit Ammoniak erfolgen. In einer bevorzugten Ausführungsform werden die Vernetzungsagenzien nach der Neutralisation zugesetzt. Weiterhin können die vernetzenden Agenzien auch zu einem späteren Zeitpunkt der Reaktion nach weitgehendem Ausdestillieren der Reaktionsmischung zu dem viskosen Polymerprodukt von Maleinsäuremonoestern und Ammoniak zugesetzt werden, gegebenenfalls unter Zusatz von sauren oder lewis-sauren Katalysatoren wie beispielsweise Titan-(IV)-alkoxiden. In beiden Ausführungsformen erfolgt eine weitere Behandlung durch Abdestillieren (von Wasser, dem kurzkettigen Alkohol R⁴OH sowie gegebenenfalls anderen Lösungsmitteln), gegebenenfalls unter vermindertem Druck, bei 70 bis 220 °C, vorzugsweise bei 110 bis 160 °C. Das Verhältnis der Dicarbonsäuremonomeren zu den Amino- bzw. Hydroxygruppen der polyfunktionellen Vernetzungsagenzien kann 99,5 : 0,5 bis 10 : 90, vorzugsweise 95 : 5 bis 50 : 50, betragen.

In einer weiteren Ausführungsform werden die polyfunktionellen Amino- bzw. Hydroxyverbindungen in einer ersten Stufe mit 0,1 bis 2,0 Äquivalenten, vorzugsweise 0,5 bis 1,2 Äquivalenten an Maleinsäureanhydrid pro Hydroxy- bzw. Aminofunktion umgesetzt. Die daraus resultierenden Maleinsäurederivate werden zu der Eduktmischung aus den Maleinsäuremonoestern (V) und (VI) vor oder während der Neutralisation mit Ammoniak zugesetzt.

Die beschriebenen Vorgehensweisen zum Zusatz der Molekularmassen erhöhenden Komponenten können beliebig kombiniert werden.

Die resultierenden Polymeren können nachbehandelt werden, beispielsweise durch Behandlung mit Ammoniak, Umesterungskatalysatoren wie beispielsweise lewissauren Titan-(IV)-Verbindungen, mit Aktivkohle oder anderen Adsorbentien, Bleichung mit Oxidationsmitteln wie H₂O₂, Cl₂, O₃, Natriumchlorit, Natriumhypochlorit etc. oder Reduktionsmitteln wie beispielsweise NaBH₄ oder H₂ in Gegenwart von Katalysatoren, unter üblichen Bedingungen.

Die erfindungsgemäßen Copolymeren zeigen im Vergleich zu den copolymeren Polyasparaginsäurederivaten, welche ohne Zusatz Molekularmassen erhöhender Gruppen hergestellt werden, eine deutliche Erhöhung der resultierenden Molekularmassen. Sie besitzen hervorragende Eigenschaften als Sequestriermittel, als Additive zu Farben und Lacken, als Schaumstabilisatoren, Tenside und Emulgatoren. Insbesondere die Temperatur- und Langzeitstabilität von O/W- und W/O-Emulsionen wird positiv beeinflußt.

Die erfindungsgemäßen Polymeren können als Emulgatoren für kosmetische Emulsionen eingesetzt werden, beispielsweise für Lotionen mit einer vergleichsweise niedrigen Viskosität oder Cremes und Salben mit einer hohen Viskosität, für Anwendungen als Hautpflegemittel wie beispielsweise Tagescremes, Nachtcremes, Pflegecremes, Nährcremes, Bodylotions, Salben und dergleichen. Als weitere Hilfs- und Zusatzstoffe können übliche Coemulgatoren, Konsistenzgeber, Ölkörper, Überfettungsmittel, Fette, Wachse, Stabilisatoren, Wirkstoffe, Glycerin, Farb- und Duftstoffe enthalten.

Als Konsistenzgeber können hydrophile Wachse, beispielsweise C₁₂-C₃₀-Fettalkohole, C₁₆-C₂₂-Fettsäuren, Glycerinmono- und -diester und Sorbitanmono- und -diester von gesättigten Fettsäuren mit 12 bis 22 C-Atomen eingesetzt werden.

Als weitere Coemulgatoren kommen beispielsweise in Frage: Anlagerungsprodukte von 2 bis 30 Mol Ethylenoxid und/oder 0 bis 5 Mol Propylenoxid an C₁₂-C₃₀-Fettalkohole und Wollwachsalkohole, vorzugsweise lineare, gesättigte C₁₆-C₂₂-Fettalkohole; Ethylenoxidanlagerungsprodukte von Glycerinmono- und -diestern und Sorbitanmono- und -diestern von gesättigten und ungesättigten Fettsäuren mit 6 bis 22 C-Atomen; Anlagerungsprodukte von 2 bis 30 Mol Ethylenoxid und/oder 0 bis 5 Mol Propylenoxid an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Anlagerungsprodukte von Ethylenoxid an Fette und Öle; Polyolester von gesättigten oder ungesättigten C₁₂-C₂₂-Fettsäuren, auch verzweigte oder hydroxysubstituierte, beispielsweise Ester von Pentaerythrit, Polyglycerin, Zuckeralkoholen wie Sorbit und Polysacchariden wie Cellulose; Polysiloxan-Polyalkyl-Polyether-Copolymere und deren Derivate sowie hydrophob modifizierte Polyasparaginsäurederivate in Frage. Als Coemulgatoren können auch anionische, kationische, amphotere oder zwitterionische Tenside sowie nichtionische Tenside, beispielsweise aus den als verträglichkeitsfördernden Agenzien beschriebenen Gruppen enthalten sein.

Es können jeweils beliebige Mischungen der obengenannten Konsistenzgeber und Coemulgataren eingesetzt werden.

Als Ölkörper kommen beispielsweise Ester von linearen C₆-C₂₀-Fettsäuren mit linearen C₆-C₂₀-Fettalkahalen, Ester von verzweigten C₆-C₁₃-Carbansäuren mit linearen C₆-C₂₀-Fettalkahalen, Ester von linearen C₆-C₂₀-Fettsäuren mit Isopropanol oder verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische beziehungsweise naphthenische Kohlenwasserstoffe in Betracht.

Als Überfettungsmittel können beispielsweise Lanolin und Lecithinderivate sowie deren Ethoxylate, Polyolfettsäureester, Monoglyceride und Fettsäurealkanolamide verwendet werden.

Es können Siliconverbindungen wie Polydimethylsiloxane, Cyclodimethicone sowie amino-, fettsäure-, alkohol-, epoxy- fluor- und/oder alkylmodifizierte Siliconverbindungen sowie Wachse wie beispielsweise Bienenwachs, Paraffinwachse oder Mikrowachse enthalten sein. Die Emulsionen können Verdickungsmittel wie Polyacrylsäurederivate oder Polysaccharide wie beispielsweise Xanthan, Carboxymethylcellulose, Hydroxyethylcellulose, kationische Cellulose- oder Stärkederivate, kationische Chitin- oder Chitosanderivate, kationische Siliconpolymere, Copolymere von Diallylammoniumsalzen beispielsweise mit Acrylamiden, Polyethylenimin enthalten. Weiterhin können anorganische Elektrolyte wie Alkali- und Erdalkalimetall- oder Ammoniumhalogenide, -sulfate, -nitrate oder -carbonate, oder Metallsalze von Fettsäuren, beispielsweise Magnesium-, Aluminium- oder Zinkstearat als Stabilisatoren, oder Zinksalze der Ricinolsäure als Geruchshemmer enthalten sein. Es können übliche Sonnenschutzwirkstoffe, Puffersubstanzen, Antioxidantien, Duftstoffe, Farbstoffe, biogene Wirkstoffe wie Pflanzenextrakte oder Vitaminkomplexe, pharmazeutische Wirkstoffe sowie übliche feuchtigkeitsregulierende Substanzen wie Pyrrolidindion-2-carboxylat und Polyhydroxyverbindungen wie Glycerin, Polyglycerine, Propandiol, Polyethylenglykole, Mono- und Polysaccharide enthalten sein.

Weiterhin können die Emulsionen Perlglanzmittel wie Ethylenglykoldistearat, feste anorganische Zusatzstoffe wie Metalloxide, Silicate, Tonmineralien etc. sowie übliche Konservierungsmittel enthalten.

Die Emulgierung kann in an sich bekannter Weise, d. h. beispielsweise durch Heiß-, Kalt-, Heiß-Heiß/Kalt- bzw. PIT-Emulgierung erfolgen.

Je nach Art der verwendeten Polyasparaginsäurederivate und Coemulgatoren sind Emulsionen vom O/W- oder vom W/O-Typ zugänglich.

In einer bevorzugten Ausführungsform der Erfindung enthalten die Emulsionen - bezogen auf den Emulgatoranteil - 1 bis 99 Gew.-%, vorzugsweise 5 bis 50 Gew.-% Polyasparaginsäurederivate, 0 bis 99 Gew.-%, in O/W-Emulsionen, vorzugsweise 15 bis 80 Gew.-% eines oder mehrerer Konsistenzgeber, sowie 0 bis 99, vorzugsweise 20 bis 75 Gew.-% weiterer Coemulgatoren. Der nichtwäßrige Anteil der Emulsionen, der sich weitestgehend aus dem Emulgator/Konsistenzgeber sowie dem Ölkörpergehalt zusammensetzt, liegt üblicherweise bei 5 bis 95 und vorzugsweise bei 15 bis 75 Gew.-%. Das bedeutet umgekehrt, daß die Emulsionen 5 bis 95 und vorzugsweise 25 bis 85 Gew.-% Wasser enthalten können, abhängig davon, ob Lotionen mit einer vergleichsweise niedrigen Viskosität oder Cremes und Salben mit einer hohen Viskosität hergestellt werden sollen.

Die erfindungsgemäßen Polyasparaginsäurederivate mit einem naturnahen Polyaminosäurerückgrat sind milde Tenside, welche alleine oder in Kombination mit anionischen, kationischen nichtionischen, zwitterionischen und/oder amphoteren Tensiden eingesetzt werden können. Es sind feste, flüssige oder pastöse Zubereitungen möglich, beispielsweise Seifenstücke, Waschlotionen, Duschgele, Shampoos.

Die in Kombination mit den erfindungsgemäßen Polyasparaginsäurederivaten in Emulsionen oder tensidischen Zubereitungen einsetzbaren Tenside können beispielsweise anionische Tenside aus der Gruppe der Sulfate, Sulfonate, Carboxylate sowie Mischungen derselben sein. Die anionischen Gruppen können in neutralisierter Form vorliegen, mit kationischen Gegenionen aus der Gruppe der Alkalimetalle, Erdalkalimetalle, Ammonium oder substituiertem Ammonium. Eingesetzt werden beispielsweise Alkylbenzolsulfonate, α-Olefinsulfonate, α-sulfonierte Fettsäureester, Fettsäureglycerinestersulfate, Paraffinsulfonate, Alkylsulfate, Alkylpolyethersulfate, Sulfobernsteinsäurealkylester, Fettsäuresalze (Seifen), Fettsäureester der Polymilchsäure, N-Acylaminosäureester, N-Acyltaurate, Acylisethionate, Ethercarboxylate, Monoalkylphosphate, N-Acylaminosäurederivate, wie N-Acylaspartate oder N-Acylglutamate, N-Acylsarcosinate, Polyasparaginsäurederivate und andere.

Die in Kombination mit den erfindungsgemäßen Polyasparaginsäurederivaten weiter einsetzbaren Tenside können beispielsweise amphotere oder zwitterionische Tenside sein, beispielsweise Alkylbetaine, Alkylamidoalkylbetaine des Typs Cocoamidopropylbetain, Sulfobetaine, Phosphobetaine, Sultaine und Amidosultaine, Imidazoliniumderivate, Amphoglycinate und andere.

Die in Kombination mit den erfindungsgemäß Polyasparaginsäurederivaten einsetzbaren Tenside können weiterhin beispielsweise nichtionische Tenside sein, beispielsweise oxethylierte Fettalkohole, oxethylierte Alkylphenole, oxethylierte Fettsäureester, oxethylierte Mono-, Di- oder Triglyceride oder Polyalkylenglykolfettsäureester. Andere nichtionische Tenside können aus der Gruppe der Alkylpolysaccharide, beispielsweise Alkyl- oder Alkenylpolyglucoside sowie deren Ethoxilierungsprodukten, Zuckerester, beispielsweise Fettsäureester der Glucose, Saccharose, Fructose oder des Methylglucosids, Sorbitolfettsäureester und Sorbitanfettsäureester (gegebenenfalls oxethyliert), Polyglycerinester, Fettsäurealkanolamide, N-Acylaminozuckerderivate beispielsweise N-Acylglucamine, langkettige tertiäre Aminoxide oder Phosphinoxide sowie Dialkylsulfoxide stammen.

Die in Kombination mit den erfindungsgemäßen Polyasparaginsäurederivaten eingesetzten Tenside können beispielsweise aus der Gruppe der quarternären Ammoniumverbindungen, quarternisierten Proteinhydrolisate, Alkylamidoamine, quarternären Esterverbindungen, quarternären Siliconöle oder quarternären Zucker- und Polysaccharidderivate ausgewählt werden.

Die in Kombination mit den erfindungsgemäßen Polyasparaginsäurederivaten eingesetzten Tenside können auch beliebige Kombinationen aus zwei oder mehr Tensiden der obengenannten Kategorien sein.

Die erfindungsgemäßen Tensidzubereitungen können weitere Hilfs- und Zusatzstoffe enthalten wie beispielsweise Wasser und Lösungsmittel beispielsweise aus der Gruppe der Alkohole und Polyole, Verdickungsmittel, Trübungsmittel, beispielsweise Glykolesterderivate; Moisturizer, Emollients wie tierische und pflanzliche Öle, Carbonsäureester, Lanolin, Bienenwachs, Silicone; polymere Agenzien zur Verbesserung des Hautgefühls, konditionierende, pflegende oder pharmazeutisch wirksame Bestandteile wie z. B. kationische oder amphotere Polymere, Proteine und Proteinderivate, Lanolinderivate, Panthothensäure, Betain, Polydimethylsiloxane oder deren Derivate, Sonnenschutzwirkstoffe sowie Lösungsvermittler, Stabilisatoren, Puffersubstanzen, Geruchsstoffe, Konservierungsmittel und/oder Farbstoffe.

Die Polyasparaginsäurederivate enthaltenden Tensidzubereitungen lassen sich vorteilhaft anwenden in beispielsweise Haarshampoo, Duschbad, Schaumbadzubereitung, Hand-, Gesichts und Intimreinigungslotion, Flüssigseife, Seifenstück, Rasiercreme, Handwaschpaste, hautfreundlichen Geschirrspülmittel, Reinigungsmittel für glatte Oberflächen sowie in Zahncreme.

Die erfindungsgemäßen Polyasparaginsäurederivate lassen sich vorteilhaft einsetzen als Dispergiermittel zur Herstellung wäßriger Pigmentpasten. Dazu werden die hydrophob modifizierten Polyasparaginsäurederivate vorteilhaft mit dem Stand der Technik entsprechenden Neutralisationsmitteln, insbesondere Aminen neutralisiert. Insbesondere bevorzugt ist hier die Verwendung von Dimethylethanolamin oder 2-Amino-2-methylpropanol. Zur Herstellung wäßriger Pigmentpasten werden 0,1 bis 100 Gew.-%, vorzugsweise 0,5 bis 50 Gew.-%, insbesondere 2 bis 15 Gew.-% bezogen auf das Gewicht der Pigmente verwendet. Die hydrophob modifizierten Polyasparaginsäurederivate können bei der erfindungsgemäßen Verwendung entweder vorab mit den zu dispergierenden Pigmenten vermischt werden oder direkt in dem Dispergiermedium (Wasser, eventuelle Glycolzusätze) vor oder gleichzeitig mit der Zugabe der Pigmente und etwaiger anderer Feststoffe gelöst werden. Die Neutralisation kann dabei vor oder während der Herstellung der Pigmentpasten erfolgen. Bevorzugt werden Polyasparaginsaurezubereitungen eingesetzt, welche bereits partiell oder vollständig neutralisiert wurden.

Die erfindungsgemäßen Polyasparaginsäurederivate können auch in beliebigen Gemischen mit weiteren, dem Stand der Technik entsprechenden Dispergieradditiven, beispielsweise aus der Gruppe der Fettsäurealkoxylate, Poly(meth)acrylate, Polyester, Polyether etc., eingesetzt werden.

Als Pigmente können in diesem Zusamenhang beispielsweise anorganische oder organische Pigmente, sowie Ruße genannt werden. Als anorganische Pigmente seien exemplarisch genannt Titandioxid und Eisenoxide. In Betracht zu ziehende organische Pigmente sind beispielsweise Azopigmente, Metallkomplex-Pigmente, Phthalocyanin-Pigmente, anthrachinoide Pigmente, polycyclische Pigmente, insbesondere solche der Thioindigo-, Chinacridon-, Dioxazin-, Pyrrolopyrrol-, Naphthalintetracarbonsäure-, Perylen-, Isoamidolin(on)-, Flavanthron-, Pyranthron- oder Isoviolanthron-Reihe. Die erfindungsgemäßen Polyasparaginsäurederivate können als Dispergiermittel beispielsweise für Lacke und Farben eingesetzt werden.

Füllstoffe, die beispielsweise in wäßrigen Lacken dispergiert werden können, sind beispielsweise solche auf Basis von Kaolin, Talkum, anderen Silikaten, Kreide, Glasfasern, Glasperlen oder Metallpulvern.

Als Lacksysteme, in denen die erfindungsgemäßen Pigmentpasten aufgelackt werden können, kommen beliebige wäßrige 1K- oder 2K-Lacke in Betracht. Beispielhaft genannt seien wäßrige 1K-Lacke wie beispielsweise solche auf Basis von Alkyd-, Acrylat-, Epoxid-, Polyvinylacetat-, Polyester- oder Polyurethanharzen oder wäßrige 2K-Lacke, beispielsweise solchen auf Basis von hydroxylgruppenhaltigen Polyacrylat- oder Polyesterharzen mit Melaminharzen oder gegebenenfalls blockierten Polyisocyanatharzen als Vernetzer. In gleicher Weise seien auch Polyepoxidharzsysteme genannt.

### Beispiele

### Vergleichsbeispiele 1 und 2

### Poly(asparaginsäure-co-alkylaspartat)

Die Herstellung der Polyasparaginsäureester erfolgte in Analogie zur DE 195 45 678 A durch Umsetzen der Edukte (Monoethylmaleat, Monoalkylmaleat, gelöst in Methylisobutylketon) mit 1,0 bis 1,5 Equivalenten an Ammoniakgas und Ausdestillieren der Reaktionsmischung i. Vak. bei 110 bis 140 °C für 4 bis 6 h.

Die Bestimmung der Veresterungsgrade erfolgte NMR-spektroskopisch, die mittleren Molekularmassen wurden durch Gelpermeationschromatographie bestimmt (Säule 2xSDV100Å/Microgel 1000Å; Tetrahydrofuran/Oxalsäure, kalibriert gegen PMMA)

| BEISPIEL | ALKYLREST | EDUKT: MOL ALKYLMALEAT | EDUKT: MOL ETHYLMALEAT | PRODUKT: MOL-% ALKYLESTER | PRODUKT: MOL-% ETHYLESTER | MOL-% SÄURE | M_{W} (GPC) |
|---|---|---|---|---|---|---|---|
| 1 | Decyl | 1,0 | 3,0 | 20 | 3 | 77 | 1200 |
| 2 | Cetyl | 1,2 | 2,8 | 27 | 6 | 67 | 1800 |

### Beispiel 3

300 g des Produktes aus Beispiel 1 wurden bei 135 °C mit 70 g 1,6-Diaminohexan gemischt und bei 140 °C 8 h unter Destillationsbedingungen gerührt.
GPC : M_{w} = 2500

### Beispiel 4

400 g des Produktes aus Beispiel 1 wurden bei 120 °C mit 86 g 1,6-Hexandiol gemischt, 4 g Tetrabutyltitanat werden zugesetzt und die Mischung wird bei 145 °C und 200 mbar 8 h ausdestilliert.
GPC : M_{w} = 3900

### Beispiel 5

Analog zu Beispiel 3 wurden 400 g des Produktes aus Bsp. 2 mit 140 g 1,6-Diaminohexan umgesetzt.
GPC : M_{w} = 9800

### Beispiel 6

340 g (1 Mol) Cetylmaleat, 286 g (2 Mol) Ethylmaleat und 79 g (0,25 Mol) des Umsetzungsproduktes aus Maleinsäureanhydrid und 1,6-Hexandiol (1 : 1) wurden in 4-Methyl-2-pentanon gelöst und mit 4 Mol Ammoniakgas behandelt. Bei 110 bis 130 °C wurde in Vakuum für 5 h ausdestilliert.
GPC : M_{w} = 6200

### Beispiele 7 bis 10

### O/W-Emulsionen mit Polyasparaginsäurederivaten

| | |
|---|---|
| Cetylpolyaspartat aus Beispiel 2, 4, 5 beziehungsweise 6 (25 % in Wasser, pH 5,5) | 2,0 % |
| Glycerin | 3,0 % |
| Konservierungsmittel | 0,1 % |
| Wasser | 70,4 % |
| Glycerinmonostearat (Tegin® M, Th. Goldschmidt AG) | 4,5 % |
| Capryl-Caprintriglycerid, (Tegosoft®CT, Th. Goldschmidt AG) | 20,0 % |

Die wäßrige Phase und die Ölkörper/Glycerinmonostearatmischung wurden bei 70 °C zusammengegeben, intensiv mit einem Rotor-Stator-Homogenisator bearbeitet (SG/220V, 2 min). Die Wasserseparation der O/W-Emulsionen wurde nach 2 d Lagerung bei 20 °C und nach weiteren 7 d Lagerung bei 45 °C bestimmt. Die sensorische Bewertung der Proben zeigte bei Bsp. 5 keine Änderung der cremeartigen Konsistenz bei der Lagerung, beim Vergleich eine spürbare Verschlechterung.

| BEISPIEL | EMULGATOR AUS BEISPIEL | WASSERSEPARATION NACH 2 TAGEN/20 °C (VOL.-%) | WASSERSEPARATION NACH 28 TAGEN/45 °C (VOL.-%) |
|---|---|---|---|
| 7 | 2 | < 0,1% | 6 |
| 8 | 4 | < 0,1 % | 1 |
| 9 | 5 | < 0,1 % | 0,5 |
| 10 | 6 | < 0,1 % | 2 |

Diese Ergebnisse zeigen die Verbesserung der Emulsionsstabilität bei den mit polyfunktionellen Agenzien modifizierten Polyasparaginsäureestern.

### Beispiel 11

### Tensidzubereitungen mit Polyasparaginsäurederivaten

| REZEPTUR | A (GEW.-%) | B (GEW.-%) |
|---|---|---|
| Poly(asparaginsäure-co-decylasparat) nach Beispiel 3, (50 %ig in Wasser, pH 5,5) | 0,0 % | 1,0 % |
| Texapon® N28 (28 % Natriumlaurylethersulfat (Henkel KGaA) | 21,4 % | 21,4 % |
| Tego® Betain F50 (37,5 % Cocamidopropylbetain, Th. Goldschmidt AG) | 16,0 | 16,0 |
| Wasser ad 100 %, pH ad 6,0 | | |

Die Schaumeigenschaften der Tensidmischung werden durch Aufschäumen einer verdünnten Tensidlösung bestimmt. (0,5 Gew.-% WAS, 8°dH, 30 °C, Ystral-Leitstrahlmischer, 750 W, 2 min)

| MISCHUNG | SCHAUMVOLUMEN [ML] | WASSERSEPARATION 10 MIN [ML] | SCHAUMDICHTE [G/ML] |
|---|---|---|---|
| A | 1490 ± 17 | 240 ± 2.0 | 0.208 ± 0.002 |
| B | 1584 ± 11 | 231 ± 2.9 | 0.193 ± 0.003 |

Dieses Beispiel belegt den positiven Einfluß der Polyasparaginsäurederivate auf das Schaumverhalten von Tensidsystemen.

### Beispiel 12

### Formulierung für ein Duschgelkonzentrat

| | |
|---|---|
| Poly(asparaginsäure-co-decylaspartat), nach Beispiel 3, (48 %ig in Wasser, pH 5,5) | 9,0 % |
| Texapon® N70 (70 % Natriumlaurylethersulfat, Henkel KGaA) | 32,0 % |
| Tagat®R40 (PEG-40-Ethoxylat von hydriertem Rizinusöl, Th. Goldschmidt AG) | 5,0 % |
| Tego® Glucosid 810 (60 % Capryl/Capringlucosid, Th. Goldschmidt AG) | 9,0 % |
| Zitronensäure (20 %) | 0,9 % |
| NaCl (25 %) | 8,5 % |
| Wasser | 17,6 % |
| Tego® Betain F50 (37,5 % Cocoamidopropylbetain,Th. Goldschmidt AG) | 18,0 % |

### Beispiel 13

### Pflegecreme auf O/W Basis

| | |
|---|---|
| Poly(asparaginsäure-co-cetylaspartat), nach Beispiel 5, (50 %ig in Wasser, pH 5,5) | 4,5 % |
| Tego® Care 450 (Polyglyceryl-3-methylglucosiddistearat, Th. Goldschmidt AG) | 1,0 % |
| Tegin® M (Glycerylstearat, Th. Goldschmidt AG) | 0,5 % |
| Tego® Alkanol 18 (Stearylalkohol, Th. Goldschmidt AG) | 0,3 % |
| Avocadoöl | 12,0 % |
| Tegosoft® CT (Capryl-Caprintriglycerid, Th. Goldschmidt AG) | 9,0 % |
| Glycerin | 3,0 % |
| Wasser | 69,7 % |
| NaOH (10 %) ad pH 5,5 | |

## Patentansprüche

1. Von Polyaminosäuren abgeleitete Copolymere, die zu mindestens 75 Mol-% der vorhandenen Einheiten aus Struktureinheiten der allgemeinen Formeln (I), (II) oder (III) 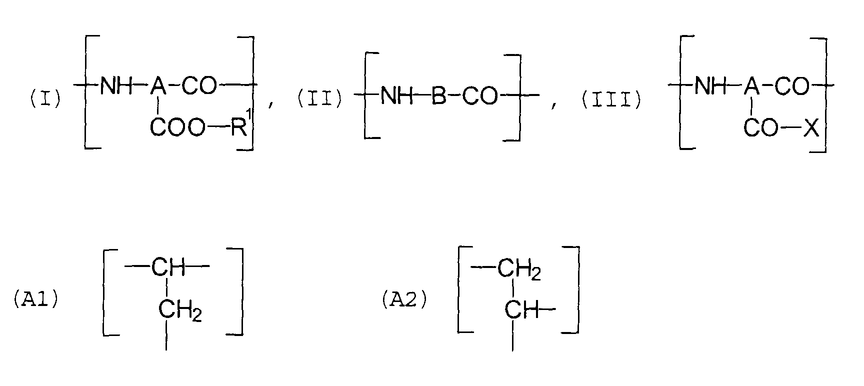 bestehen, in denen die Strukturelemente A gleiche oder verschiedene trifunktionelle Kohlenwasserstoftradikale mit 2 C-Atomen des Typs (A1) oder (A2) sind, wobei ein Copolymeres aus mindestens drei Einheiten der Formel (I) besteht, worin
R¹ die Bedeutung von R², R³ oder R⁴ hat, wobei
R² ein oder mehrere Reste aus der Gruppe der Alkali, Erdalkalimetalle, Wasserstoff oder Ammonium, [NR⁵R⁶R⁷R⁸]⁺ sind, worin R⁵ bis R⁸ unabhängig voneinander Wasserstoff, Alkyl oder Alkylen mit 1 bis 22 C-Atomen oder Hydroxyalkyl mit 1 bis 22 C-Atomen mit 1 bis 6 Hydroxygruppen und/oder deren Acylierungsprodukte mit C₁- bis C₂₂-Carbonsäureresten bedeuten,
R³ gleiche oder verschiedene, geradkettige oder verzweigte, gesättigte oder ungesättigte Alkyl- oder Alkenylreste R⁹ mit 6 bis 30 C-Atomen oder Radikale der Struktur -Y-R⁹ sind, wobei Y eine Oligo- oder Polyoxyalkylenkette mit 1 bis 100 Oxyalkyleneinheiten ist,
R⁴ gleiche oder verschiedene, geradkettige oder verzweigte, gesättigte oder ungesättigte Alkyl- oder Alkenylreste mit 1 bis 5 C-Atomen sind,
die Einheiten der Formel (II) für proteinogene oder nicht proteinogene Aminosäuren stehen und zu nicht mehr als 20 Gew.-% enthalten sind und
X in der Formel (III) für ein oder mehrere di- oder polyfunktionelle Radikale abgeleitet von Molekularmassen erhöhenden Agenzien, insbesondere einer Di- oder Polyhydroxyverbindung, einer Di- oder Polyaminoverbindung, oder Aminoalkoholen, mit einem linearen, verzweigten oder cyclischen, gesättigten, ungesättigten oder aromatischen Kohlenwasserstoffgerüst, gegebenenfalls oxo- oder azasubstituiert mit O- oder N-Atomen in der Kette, steht,
und mindestens jeweils ein Rest R¹ die Bedeutung von R² und mindestens ein Rest R¹ die von R³ und wenigstens ein Rest R¹ die von X annehmen muß.

2. Copolymere nach Anspruch 1, in denen mindestens ein Rest R¹ die Bedeutung von R⁴ hat.

3. Copolymere nach den Ansprüchen 1 und 2, in denen R³ gleiche oder verschiedene, geradkettige oder verzweigte, gesättigte oder ungesättigte Alkyl- oder Alkenylreste mit 8 bis 24 C-Atomen sind.

4. Copolymere nach einem der Ansprüche 1 bis 3, enthaltend als Molekularmassen erhöhende Agenzien Di- oder Polyhydroxyverbindungen, Di- oder Polyaminoverbindungen, oder Aminoalkohole oder Mischungen, mit einem linearen, verzweigten oder cycischen, gesättigten, ungesättigten oder aromatischen Kohlenwasserstoffgerüst, gegebenenfalls oxo- oder azasubstituiert mit O- oder N-Atomen in der Kette.

5. Copolymere nach einem der Ansprüche 1 bis 4, enthaltend als molekularmassenerhöhende Agenzien lineare 1,ω-Alkandiole, Glycerin, Sorbitol, 1,2-Propylenglykol, lineare 1,ω-Diaminoalkane, Lysin, Ethanolamin, Diethanolamin, Triethanolamin, Zuckerderivate, Oligo- und Polysaccharide, die Anlagerungsprodukte von Ethylenoxid und/oder Propylenoxid an die genannten Verbindungen, Polyvinylalkohol, Oligo- und Polyethylenglykole und/oder Ethylenoxid-Propylenoxid-Copolymere.

6. Verfahren zur Herstellung der Copolymeren gemäß einem der Ansprüche 1 bis 5, dadurch gekennzeichnet, daß man Ester α,β-ungesättigter Dicarbonsäuren oder deren Ammoniumsalze, insbesondere Maleinsäurederivate der allgemeinen Formeln (V) und (VI) 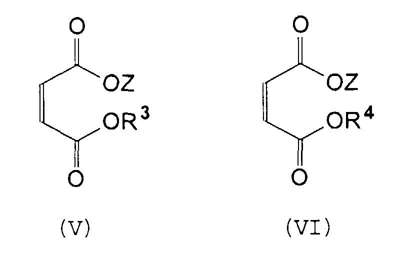 alleine oder im Gemisch miteinander, mit Ammoniak umsetzt und in das Polymer überführt und anschließend mit Molekularmassen erhöhenden Agenzien behandelt, in denen Z für Wasserstoff und/oder Ammonium steht, und R³ und R⁴ für die oben genannten Reste stehen, gegebenenfalls in Gegenwart von bis zu 20 Gew.-% proteinogener oder nicht proteinogener Aminosäuren oder deren Derivate gemäß der allgemeinen Formel (II), sowie gegebenenfalls in weiteren Schritten durch Hydrolyse Gruppen der Struktur der Formel (I), wobei R¹ die Bedeutung von R², mit der oben genannten Definition von R², hat, erzeugt.

7. Verfahren gemäß Anspruch 6, dadurch gekennzeichnet, daß man Ester α,β-ungesättigter Dicarbonsäuren oder deren Ammoniumsalze, insbesondere Maleinsäurederivate der allgemeinen Formeln (V) und (VI) in Gegenwart von Molekularmassen erhöhenden Agenzien mit Ammoniak umsetzt und in das Polymer überführt.

8. Kosmetische Emulsionen, enthaltend Copolymere nach einem der Ansprüche 1 bis 5, dadurch gekennzeichnet, daß der nichtwäßrige Anteil 5 bis 99 Gew.-% an Ölkörpern aus der Gruppe der Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₂₀-Fettsäuren mit verzweigten Alkoholen, Ester von linearen und/oder verzweigten C₆-C₂₀-Carbonsäuren mit mehrwertigen Alkoholen und/oder Guerbetalkoholen, Triglyceride auf Basis von C₆-C₁₀-Fettsäuren, pflanzliche und tierische Öle und Fette, verzweigte primäre Alkohole, substituierte Cyclohexane, Guerbetcarbonate, Dialkylether und/oder aliphatische bzw. naphthenische Kohlenwasserstoffe enthält.

9. Kosmetische Emulsionen nach Anspruch 8, enthaltend hydrophile Wachse aus der Gruppe der C₁₂-C₃₀-Fettalkohole, Wollwachsalkohole, C₁₆-C₂₂-Fettsäuren, Glycerinmono- und -diester und Sorbitanmono- und -diester von gesättigten Fettsäuren mit 12 bis 22 C-Atomen.

10. Kosmetische Emulsionen nach Anspruch 8 oder 9, enthaltend ein oder mehrere Coemulgatoren aus der Gruppe der Anlagerungsprodukte von Ethylenoxid oder Ethylenoxid und Propylenoxid an C₁₂-C₃₀-Fettalkohole und Wollwachsalkohole, der Ethylenoxidanlagerungsprodukte von Glycerinmono- und diestern und Sorbitanmono- und -diestern von gesättigten und ungesättigten Fettsäuren mit 6 bis 22 C-Atomen, der Anlagerungsprodukte von Ethylenoxid und/oder Propylenoxid an Fettsäuren mit 12 bis 22 C-Atomen und an Alkylphenole mit 8 bis 15 C-Atomen in der Alkylgruppe, der C₁₂-C₁₈-Fettsäuremono- und -diester von Anlagerungsprodukten von Ethylenoxid an Glycerin, der Anlagerungsprodukte von Ethylenoxid an Fette und Öle, der Polyolester von gesättigten oder ungesättigten C₁₂-C₂₂-Fettsäuren, auch verzweigte oder hydroxysubstituierte, der Polysiloxan-Polyalkyl-Polyether-Copolymere und deren Derivate, der anionischen Tenside, kationischen Tenside, nichtionischen Tenside sowie zwitterionischen oder amphoteren Tenside.

11. Verwendung der Emulsionen nach einem der Ansprüche 8 bis 10 als Hautpflegemittel, Tagesoreme, Nachtcreme, Pflegecreme, Nährcreme, Bodylotion, pharmazeutische Salbe und Lotion, Aftershavelotion und Sonnenschutzmittel.

12. Verwendung der Copolymeren nach einem der Anprüche 1 bis 5 in tensidischen Zubereitungen für Reinigungsmittel und/oder kosmetische Mittel, enthaltend Polyasparaginsäurederivate, oder Polyasparaginsäurederivate und ein oder mehrere weitere Tenside aus der Gruppe der anionischen, kationischen, nichtionischen, amphoteren und zwitterionischen Tenside sowie deren Mischungen daraus neben üblichen Hilfs- und Zusatzstoffen.

13. Verwendung der Tensidzubereitungen nach Anspruch 12 als Shampoo, Waschlotion und Reinigungsmittel für Gesicht, Haar, Haut und Intimbereich, Rasiercreme und -lotion, Flüssigseife, Geschirrspülmittel, Reinigungsmittel für glatte Oberflächen, Seifenstück, Schaumbad, Duschgel sowie in Zahncreme und/oder Mundspülung.

14. Verwendung der Copolymeren gemäß einem der Ansprüche 1 bis 5 als Waschmittelhilfsstoff, Komplexagens für mehrwertige Kationen, Dispergierhilfsmittel für Lacke und Farben, Inkrustationsinhibitor, Absorbermaterialien, Metalldesaktivator in Kunststoffen, als Hilfsstoff in der Papier-, Leder- und Textilindustrie oder als wirkungsverstärkender Zusatzstoff zu Pestiziden oder Insektiziden.
